# EUROPÄISCHE PATENTANMELDUNG

(11) **EP 1 281 957 A1**
(43) Veröffentlichungstag der Anmeldung: **05.02.2003**
(21) Anmeldenummer: 01118235.9
(22) Anmeldetag: 30.07.2001
(51) Int. Cl.: G01N 33/00

(54) **Melder für brennbare Gase mit einer Einrichtung zur Selbstüberwachung**

(71) Anmelder: Siemens Building Technologies AG, 8034 Zürich (CH)
(72) Erfinder: Pleisch, Rolf, 8604 Volketswil (CH); Forster, Martin, Dr., 8645 Jona (CH); Hold, Albert, 8708 Männedorf (CH)
(74) Vertreter: Dittrich, Horst, Dr.

(57) **Zusammenfassung**

Der Melder enthält einen in einem Gehäuse (2) angeordneten Sensor (5) und eine Einrichtung (9) zur automatischen Überprüfung der Empfindlichkeit des Sensors (5). Die genannte Einrichtung (9) ist durch im Inneren des Gehäuses (2) angeordnete Mittel zur Speicherung einer Testsubstanz und zur gesteuerten Freisetzung einer definierten Menge von dieser gebildet. Die Testsubstanz ist durch ein festes Kohlenhydrat oder einen flüssigen Kohlenwasserstoff gebildet, die Freisetzung erfolgt durch Erhitzen.

Die genannten Mittel (9) umfassen ein Substrat mit einer Mehrzahl von individuell freisetzbaren Portionen der Testsubstanz oder ein Flüssigkeitsreservoir, aus dem die benötigte Menge der Testsubstanz über eine geeignete Fördereinrichtung entnehmbar ist.

## Beschreibung

Die vorliegende Erfindung betrifft einen Melder für brennbare Gase, mit einem in einem Gehäuse angeordneten Sensor und mit einer Einrichtung zur automatischen Überprüfung der Empfindlichkeit des Sensors.
Gasmelder für die Detektion brennbarer Gase müssen im Feld unter oft schwierigen Umgebungsbedingungen mit Prüfgasen getestet werden, wobei ein becherförmiger Körper über den Sensor gestülpt, dieser mit dem Prüfgas begast und die Zeit bis zum Ansprechen des Melders und dessen Empfindlichkeit gemessen wird. Damit die Funktionalität der Gaswarnanlage garantiert werden kann, sind solche Prüfungen durch Normen und Richtlinien in regelmässigen Abständen von beispielsweise sechs Monaten vorgeschrieben. Dieses heute übliche Verfahren weist insbesondere die folgenden Nachteile auf:
- Sehr hohe Kosten für die Prüfgase und den Arbeitsaufwand;
- erschwerte Ersatzteillogistik, da bei der Prüfung nicht von vornherein bekannt ist, welche Sensoren die Prüfung nicht erfüllen werden;
- Unsicherheit beim Kunden bezüglich der Funktionalität der Gaswarnanlage in der Zeit zwischen den Prüfungen.

Wegen dieser Nachteile wird vom Markt in zunehmendem Mass eine automatische Selbstüberwachung der Gassensoren gefordert, insbesondere bei Pellistorsensoren, welche durch verschiedenste Substanzen (Katalysatorgifte wie Silikon-, Halogen-, Blei- oder Schwefelverbindungen) beeinträchtigt oder beschädigt werden können. Existierende automatische Selbstüberwachungssysteme bestehen aus einem Leitungssystem zwischen Druckflaschen mit Prüfgasen und den zu überprüfenden Sensoren, wobei die Leitungen zeitgesteuerte Ventile aufweisen. Da diese Systeme wegen der erforderlichen Installationen relativ teuer und durch die zusätzlichen Leitungen und die Druckflaschen ausserdem ästhetisch nicht ansprechend sind, konnten sie sich bisher für die Mehrheit der möglichen Anwendungen nicht durchsetzen.
Durch die Erfindung soll nun eine Einrichtung zur automatischen Überprüfung der Empfindlichkeit von Sensoren für brennbare Gase angegeben werden, welche keine aufwändigen und kostspieligen Installationen erfordert.
Die erfindungsgemässe Lösung der gestellten Aufgabe ist dadurch gekennzeichnet, dass die genannte Einrichtung durch im Inneren des Gehäuses angeordnete Mittel zur Speicherung einer Testsubstanz und zur gesteuerten Freisetzung einer definierten Menge von dieser gebildet ist.
Bei der erfindungsgemässen Einrichtung sind also keinerlei externe Installationen erforderlich, da das Prüfgas nicht in einer externen Druckflasche sondern im Melderinneren gespeichert ist und somit weder Druckflaschen, noch Leitungen zu diesen, noch gesteuerte Ventile in den Leitungen nötig sind.
Eine erste bevorzugte Ausführungsform des erfindungsgemässen Melders ist dadurch gekennzeichnet, dass die Testsubstanz durch ein festes Kohlenhydrat oder einen flüssigen Kohlenwasserstoff gebildet ist, und dass die Freisetzung durch Erhitzen erfolgt.
Eine zweite bevorzugte Ausführungsform des erfindungsgemässen Melders ist dadurch gekennzeichnet, dass die Testsubstanz bei der Freisetzung auf ihren Verdampfungspunkt erhitzt wird.
Eine dritte bevorzugte Ausführungsform des erfindungsgemässen Melders ist dadurch gekennzeichnet, dass die genannten Mittel ein Substrat mit einer Mehrzahl von individuell freisetzbaren Portionen der Testsubstanz umfassen.
Eine vierte bevorzugte Ausführungsform ist dadurch gekennzeichnet, dass das Substrat sandwichartig aufgebaut ist und eine matrixartige Anordnung der genannten Portionen enthält. Vorzugsweise ist jedem Speicherplatz der Matrix mindestens ein individuell ansteuerbares Heizelement zugeordnet.
Eine weitere bevorzugte Ausführungsform des erfindungsgemässen Melders ist dadurch gekennzeichnet, dass bei Verwendung einer flüssigen Testsubstanz die einzelnen Portionen im Substrat hermetisch eingeschlossen sind und bei Erhitzung durch Aufsprengen einer Sollbruchstelle aus ihrem Speicherplatz austreten. Vorzugsweise ist die Sollbruchstelle anhand der Gitterstruktur des Substrats festgelegt.
Eine weitere bevorzugte Ausführungsform des erfindungsgemässen Melders ist dadurch gekennzeichnet, dass das Gehäuse nach aussen durch eine Diffusionssperre abgeschlossen ist, und dass die genannten Mittel im Sensorvorraum zwischen der Diffusionssperre und dem Sensor angeordnet sind.
Eine weitere bevorzugte Ausführungsform des erfindungsgemässen Melders ist dadurch gekennzeichnet, dass durch Untersuchung des Verlaufs des Sensorsignals bei Freisetzung der definierten Menge der Testsubstanz eine Überprüfung der Durchlässigkeit der Diffusionssperre erfolgt.
Bei Freisetzung der definierten Gas- oder Dampfmenge wird das Sensorsignal rasch ansteigen und nach Erreichen eines Maximalwerts wieder relativ rasch abfallen, weil ein Teil der freigesetzten Gas- bzw. Dampfmenge durch die Diffusionssperre nach aussen entweicht. Bei ganz oder teilweise verstopfter Diffusionsperre wird hingegen das Sensorsignal nur sehr langsam abklingen. Damit eröffnet sich die Möglichkeit, in einem einzigen Prüfvorgang und mit einer einzigen Prüfeinrichtung nicht nur die Empfindlichkeit des Sensors sondern auch die Durchlässigkeit der Diffusionssperre zu überprüfen.
Eine weitere bevorzugte Ausführungsform des erfindungsgemässen Melders ist dadurch gekennzeichnet, dass die genannten Mittel ein Flüssigkeitsreservoir umfassen, aus welchem die benötigte Menge der Testsubstanz über eine geeignete Fördereinrichtung entnehmbar ist. Vorzugsweise wird die Testsubstanz durch die Fördereinrichtung in den unmittelbar vor dem Gassensor liegenden Sensorvorraum gefördert.
Im folgenden wird die Erfindung anhand von Ausführungsbeispielen und der Zeichnungen näher erläutert; es zeigt:
- Fig. 1: einen Schnitt durch das Sensorgehäuse eines Gasmelders,
- Fig. 2: eine Ansicht eine ersten Ausführungsbeispiels eines Mittels zur Speicherung einer Testsubstanz,
- Fig. 3: ein Ausschnitt aus Fig. 2,
- Fig. 4: eine Variante von Fig. 3; und
- Fig. 5: eine schematische Darstellung des Sensorgehäuses mit einem zweiten Ausführungsbeispiel eines Mittels zur Speicherung einer Testsubstanz.

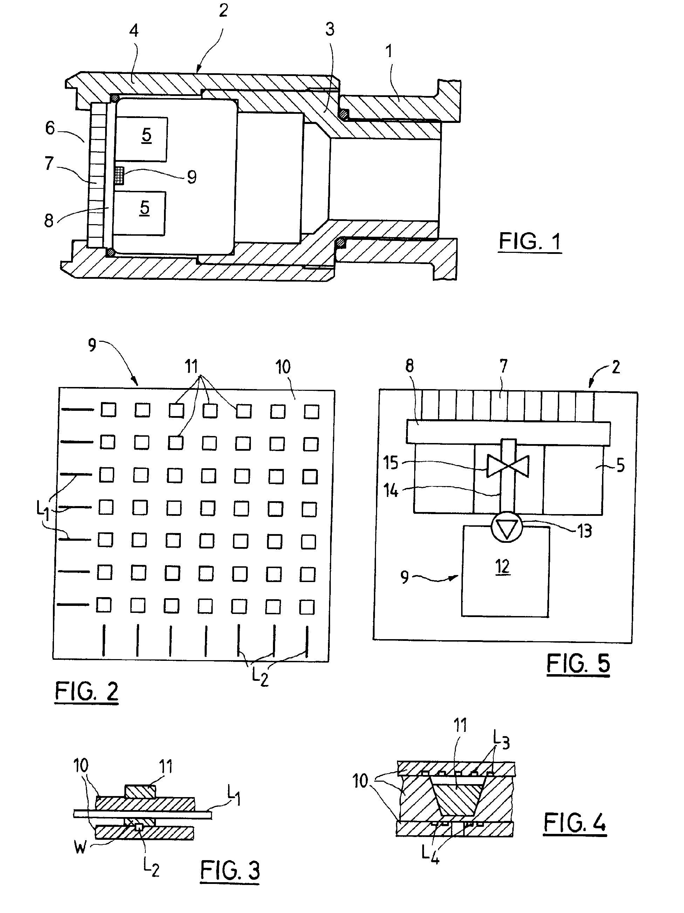

Fig. 1 zeigt einen schematischen Schnitt durch das Sensorgehäuse eines Gasmelders, welcher ein nicht dargestelltes, druckfestes Meldergehäuse aus Aluminium aufweist, in welchem die Melderelektronik eingebaut ist und welches einen Stutzen 1 aufweist, in welchen das Sensorgehäuse 2 eingeschraubt ist. Das Sensorgehäuse 2, weist ein in den Stutzen 1 geschraubtes Unterteil 3 und ein mit diesem verschraubtes Oberteil 4 auf, welch letzteres auch als Schutzkappe bezeichnet wird. Zwischen dem Unterteil 3 und dem Oberteil 4 ist ein Pellistorsensor 5 zum Nachweis von brennbaren oder explosiven Gasen angeordnet.
Der Gasmelder als solcher und auch der Sensor 5 bilden nicht Gegenstand der vorliegenden Anmeldung und sind daher hier nicht näher beschrieben. Es wird in diesem Zusammenhang auf die Gasmelder der Reihe CerGas DP60 und DP61 der Siemens Building Technologies AG, Fire & Security Products (früher Cerberus AG) verwiesen (CerGas ist ein registriertes Warenzeichen der Siemens Building Technologies AG).
Das Gehäuseoberteil 4 weist an seiner vom Gasmelder wegragenden Stirnfläche eine Öffnung 6 zur Ermöglichung des Zutritts von Gas aus dem Aussenraum zum Gassensor 5 auf. Die Öffnung 6 ist durch eine Diffusionssperre 7 zur Verhinderung des Überschlagens von Flammen aus dem Innenraum des Sensorgehäuses 2 nach aussen abgedeckt. Mit dem Bezugszeichen 8 ist der Sensorvorraum zwischen der Diffusionssperre 7 und dem Gassensor 5 bezeichnet. Im Sensorvorraum 8 ist eine in der Figur schematisch angedeutete Einrichtung 9 zur automatischen Überprüfung der Empfindlichkeit des Gassensors 5 angeordnet. Diese Einrichtung, welche den eigentlichen Kern der vorliegenden Erfindung bildet und durch Mittel zur Speicherung einer Testsubstanz und zur gesteuerten Freisetzung einer definierten Menge von dieser gebildet ist, wird nachfolgend anhand der Figuren 2 bis 5 detailliert beschrieben.
Gemäss Fig. 2 enthält die Einrichtung 9 ein Substrat 10 aus Si, SiO₂, Al₂O₃, oder einem anderen geeigneten Material mit einer Mehrzahl von Portionen 11 der Testsubstanz. Die Portionen 11 sind auf dem Substrat 10 in Matrixform angeordnet, und jeder Zeile und jeder Reihe der Matrix ist ein individuell ansteuerbarer Leiter L₁, L₂ zugeordnet. Das Substrat 10 hat beispielsweise eine Fläche von 6 mal 6 mm² und trägt 49 Portionen 11.
Fig. 3 zeigt einen vergrösserten Schnitt durch das Substrat 10 am Ort einer Portion 11. Darstellungsgemäss besteht das Substrat 10 aus zwei Plättchen, zwischen denen die Leiter L₁, L₂ verlaufen und von denen eine an ihrer Aussenfläche die Portionen 11 trägt. Ausserdem ist an jedem Kreuzungspunkt der Leiter L₁, L₂ ein Widerstand W vorgesehen, welcher ein individuell ansteuerbares Heizelement für die ebenfalls an diesem Kreuzungspunkt angeordnete Portion 11 dient. Das die Portionen 11 tragende Plättchen kann beispielsweise in Siebdrucktechnik hergestellt sein. Die Testsubstanz ist beispielsweise ein festes Kohlenhydrat, aus dem durch eine thermische Zersetzungsreaktion CO erzeugt wird.
Bei der in Fig. 4 dargestellten Variante ist die Testsubstanz durch eine brennbare Flüssigkeit gebildet, deren einzelne Portionen 11 im Substrat 10 hermetisch eingeschlossen sind. Das Substrat 10 ist sandwichartig aufgebaut und besteht aus drei Schichten, von denen die mittlere die grösste Dicke aufweist und mit Vertiefungen zur Aufnahme der Portionen 11 versehen ist. Die flüssigen Portionen 11 können mit Gelen oder in einer Matrix aus porösem Material fixiert sein.
Die Portionen werden durch eine Heizung L₃, L₄ auf ihren Verdampfungspunkt erhitzt und freigesetzt, wobei die Freisetzung durch gezielte elektrische Erwärmung (Ausdehnung, Druck) oder eventuell auch Aufsprengen einer Sollbruchstelle mittels anfänglicher Elektrolyse (Gaserzeugung) im Lösungsmittel oder in einer Mischung Lösungsmittel und Wasser erfolgt. Art und Form der Aufsprengung kann durch Ausnützung des Kristallgitters im Substrat in einer bestimmten Vorzugsrichtung bestimmt werden.
Wenn man von einer maximalen Sensorlebensdauer von 5 Jahren ausgeht und alle 6 Wochen einen Selbsttest des Sensors wünscht, müssen etwa 45 Einzelportionen 11 zur Verfügung stehen. Für eine leicht messbare Konzentration werden pro Portion 11 etwa 40 nl benötigt. Geeignete Flüssigkeiten sind Ethanol, Hexan, Heptan oder Isopropanol. Zum Ausgleich des Unterschieds zwischen der Aussentemperatur und der Temperatur im Inneren des Sensors können die Leiter L₃ an der einen Seite des Substrats 10 als Vorheizung und die Leiter L₄ an der anderen Seite des Substrats als eigentliche Heizung für die Freisetzung der Portionen 11 verwendet werden.
Die Heizungen L₁, L₂ (Fig. 3) und L₃, L₄ (Fig. 4) sind an die Stromversorgung des Melders angeschlossen und werden von der Melderelektronik gesteuert, durch welche das periodische Freisetzen der Portionen 11 der Testsubstanz erfolgt. Sobald eine Portion 11 freigesetzt wird, entweicht das Testgas in den Sensorvorraum 8 (Fig. 1) und gelangt von dort einerseits zum Gassensor 5 und andererseits durch die Diffusionssperre 7 nach aussen. Der Gassensor wird also im Normalfall, das heisst bei normaler Durchlässigkeit der Diffusionssperre, einen Anstieg der Konzentration bis zu einem Maximum und anschliessend - wegen des Entweichens eines Teils des Testgases durch die Diffusionsperre 7 - ein relativ rasches Absinken der Konzentration registrieren. Wenn jedoch die Diffusionssperre 7 ganz oder teilweise verstopft ist, wird das Absinken der Konzentration nach dem Maximum wesentlich langsamer erfolgen, was durch Abtastung des Sensorsignals in Intervallen von einigen Sekunden festgestellt werden kann. Auf diese Weise kann gleichzeitig mit der Empfindlichkeit des Sensors auch die Durchlässigkeit der Diffusionssperre 7 überprüft werden. Bei Nichterfüllen eines dieser beiden Kriterien gibt das betreffende Gaswarnsystem eine Störungsmeldung ab, welche darauf hinweist, dass ein baldiger Wechsel des Sensors und/oder der Diffusionssperre nötig ist.
Fig. 5 zeigt einen schematischen Schnitt durch ein Sensorgehäuse 2 mit einem weiteren Ausführungsbeispiel der Einrichtung zur automatischen Überprüfung der Empfindlichkeit des Gassensors. Diese Einrichtung unterscheidet sich von der in den Fig. 1 bis 4 dargestellten dadurch, dass die Testsubstanz nicht in Einzelportionen sondern in einem Reservoir gespeichert und aus diesem über eine geeignete Fördereinrichtung entnehmbar ist.
Darstellungsgemäss ist an der vom Sensorvorraum 8 abgewandten Seite des Gassensors 5 ein abgeschlossenes Flüssigkeitsreservoir 12 vorgesehen, welches die flüssige Testsubstanz enthält. Vom Flüssigkeitsreservoir 12 ist eine Pumpe 13 angeordnet, von welcher eine Leitung 14 zum Sensorvorraum 7 führt. Die Leitung 14 enthält ein Ventil 15. Die Pumpe 13, welche beispielsweise eine piezoelektrische Mikropumpe oder eine Pumpe der bei Tintenstrahldruckern verwendeten Art ist, fördert bei jedem Testvorgang die benötigte Menge an Testsubstanz vom Reservoir 12 in den Sensorvorraum 8.

## Patentansprüche

1. Melder für brennbare Gase, mit einem in einem Gehäuse (2) angeordneten Sensor (5) und mit einer Einrichtung (9) zur automatischen Überprüfung der Empfindlichkeit des Sensors (5), **dadurch gekennzeichnet, dass** die genannte Einrichtung (9) durch im Inneren des Gehäuses (2) angeordnete Mittel zur Speicherung einer Testsubstanz und zur gesteuerten Freisetzung einer definierten Menge von dieser gebildet ist.

2. Melder nach Anspruch 1, **dadurch gekennzeichnet, dass** die Testsubstanz durch ein festes Kohlenhydrat oder einen flüssigen Kohlenwasserstoff gebildet ist, und dass die Freisetzung durch Erhitzen erfolgt.

3. Melder nach Anspruch 2, **dadurch gekennzeichnet, dass** die Testsubstanz bei der Freisetzung auf ihren Verdampfungspunkt erhitzt wird.

4. Melder nach Anspruch 3, **dadurch gekennzeichnet, dass** die genannten Mittel (9) ein Substrat (10) mit einer Mehrzahl von individuell freisetzbaren Portionen (11) der Testsubstanz umfassen.

5. Melder nach Anspruch 4, **dadurch gekennzeichnet, dass** das Substrat (10) sandwichartig aufgebaut ist und eine matrixartige Anordnung der genannten Portionen (11) enthält.

6. Melder nach Anspruch 5, **dadurch gekennzeichnet, dass** jedem Speicherplatz der Matrix mindestens ein individuell ansteuerbares Heizelement (W) zugeordnet ist.

7. Melder nach Anspruch 6, **dadurch gekennzeichnet, dass** bei Verwendung einer flüssigen Testsubstanz die einzelnen Portionen (11) im Substrat (10) hermetisch eingeschlossen sind und bei der Erhitzung durch Aufsprengen einer Sollbruchstelle aus ihrem Speicherplatz austreten.

8. Melder nach Anspruch 7, **dadurch gekennzeichnet, dass** die Sollbruchstelle anhand der Gitterstruktur des Substrats (10) festgelegt ist.

9. Melder nach einem der Ansprüche 1 bis 8, **dadurch gekennzeichnet, dass** das Gehäuse (2) nach aussen durch eine Diffusionssperre (7) abgeschlossen ist und dass die genannten Mittel (9) im Sensorvorraum (8) zwischen der Diffusionssperre (7) und dem Sensor (5) angeordnet sind.

10. Melder nach Anspruch 9, **dadurch gekennzeichnet, dass** durch Untersuchung des Verlaufs des Sensorsignals bei der Freisetzung der definierten Menge der Testsubstanz eine Überprüfung der Durchlässigkeit der Diffusionssperre (7) erfolgt.

11. Melder nach Anspruch 1, **dadurch gekennzeichnet, dass** die genannten Mittel (9) ein Flüssigkeitsreservoir (12) umfassen, aus welchem die benötigte Menge der Testsubstanz über eine geeignete Fördereinrichtung (13-15) entnehmbar ist.

12. Melder nach Anspruch 11, **dadurch gekennzeichnet, dass** die Testsubstanz durch die Fördereinrichtung (13-15) in den unmittelbar vor dem Gassensor (5) liegenden Sensorvorraum (8) gefördert wird.
